# EUROPEAN PATENT APPLICATION

(11) **EP 4 629 144 A1**
(43) Date of publication of application: **08.10.2025**
(21) Application number: 22967193.8
(22) Date of filing: 30.11.2022
(51) Int. Cl.: G06N 20/00

(54) **PREDICTION DEVICE, PREDICTION METHOD, AND PREDICTION PROGRAM**

(71) Applicant: Tres Alchemix Co., Ltd., Fukuoka-shi, Fukuoka 812-0011 (JP)
(72) Inventor: YAMAZAKI Ken, Nara-shi, Nara 630-8113 (JP)
(74) Representative: AWA Sweden AB
(86) International application number: PCT/JP2022/044294
(87) International publication number: WO 2024/116360

(57) **Abstract**

A prediction device that accurately and efficiently predicts drug discovery of desired drugs as well as efficacy and side effects of drugs by integrating chemical substance information of compounds, information acquired at the time of administration to the cells, and biological or clinical information. The prediction device has an acquisition unit that acquires chemical substance information and pharmacological information of the drug; an estimation unit that estimates estimated information of the drug by performing machine learning using the chemical substance information and pharmacological information; and an output unit that predicts and output both efficacy and side effects of the drug on an organism by retraining a model of the machine learning on the basis of the estimated information.

## Description

### BACKGROUND OF THE INVENTION

### 1. Field of the Invention

This invention relates to a prediction device, a prediction method, and a prediction program. More specifically, it relates to a device, method, and program for predicting a drug that will exhibit optimal efficacy as well as the efficacy itself and other pharmacological properties by performing machine learning on the basis of the properties of the drug.

### 2. Description of Related Art

In conventional drug discovery methods, developers predict and generate effective drug compounds mainly on the basis of past knowledge and experience, and evaluate the efficacy and side effects of the drug compounds through experimental validation. However, with the conventional methods, the success or failure of drug discovery depends on the skills and proficiency of the developers, and moreover, it often depends on accidental discoveries. As a result, the success rate of new drug development is considerably low, and it takes an enormous amount of costs and time to discover a single new drug.

Artificial intelligence (AI)-based drug discovery methods have been attracting attention to reduce the enormous development costs of conventional drug discovery in recent years. As an example, the molecular docking method is used to predict the binding affinity between a target protein and a drug candidate compound, and a method to predict the optimal drug compound with the highest degree of binding has been proposed. However, such methods are only predictive if the three-dimensional structure of the target protein and the drug-binding site of the protein on the basis of it are accurately identified. Accordingly, the method cannot be applied to proteins whose three-dimensional structures have not been accurately identified, such as drug transporters and other membrane proteins. In addition, the values that can be acquired from the method correspond to prediction values of the binding affinity with a particular protein. Accordingly, additional experiments are needed to determine in what way the binding contributes to biological activity.

AI-based drug discovery methods focusing on the bioactivity of drugs, i.e., drug discovery methods that directly predict quantitative structure-activity relationships, have been under investigation for many years. However, existing methods focus only on pharmacological activity for specific proteins, and their use is limited to the search for drugs that are structurally similar to existing drugs with known efficacy. This is because in vitro data are essential to actually ascertain pharmacological activity or biological efficacy, and an astronomical amount of experimentation is needed to evaluate every compound.

As examples of drug discovery methods using artificial intelligence (AI), a method that graphs artificial compound data containing molecular structure data and uses a multilayer neural network (see patent document 1, for example), and a method that predicts the binding affinity between a biopolymer and a compound of interest based on their three-dimensional structures through machine learning (see patent document 2, for example) have been proposed. However, the acquired data only provide a partial view on the link between the compound information and its mechanisms of action in the organism.

### Prior Art Documents

### Patent Documents

Patent Document 1: Japanese Laid-Open Patent Publication No. 2020-9203
Patent Document 2: Japanese Laid-Open Patent Publication No. 2019-28879

### SUMMARY OF THE INVENTION

### Problems to be Solved by the Invention

Subsequently, the applicants have deeply considered integrating the chemical information of the compound, information on the effects at the cellular level, and information on the biological or clinical situation in predicting the structure of the desired drug.

In view of the points described above, the present invention provides a prediction device, a prediction method, and a prediction program for more accurate and efficient drug discovery of a desired drug as well as for predicting the efficacy and side effects of the drug by integrating chemical information of a compound, effect information at the cellular level, and biological or clinical information.

### Means to Solve the Problems

That is, a prediction device according to an embodiment is characterized by including an acquisition unit that acquires chemical substance information of a drug and pharmacological information of the drug; an estimation unit that estimates estimated information of the drug by performing machine learning using the chemical substance information and the pharmacological information; and an output unit that predicts and outputs both efficacy and side effect of the drug on an organism by retraining a model of the machine learning on the basis of the estimated information.

Further, the acquisition unit of the prediction device may further acquire cellular level biological information of the drug, and the estimation unit may perform the machine learning using the chemical substance information, the pharmacological information, and the cellular level biological information. Moreover, the estimation unit may estimate characteristic information of the drug on the basis of a chemical structure included in the chemical substance information and drug administration information included in the pharmacological information.

Furthermore, the estimation unit may estimate pharmacokinetic information of the drug on the basis of a chemical structure included in the chemical substance information and drug administration information included in the pharmacological information. Further, the estimation unit may estimate the biological information on the basis of a chemical structure included in the chemical substance information and drug administration information included in the pharmacological information.

Moreover, the prediction device may include a preliminary estimation unit that preliminarily estimates the chemical structure of the drug on the basis of the characteristic information of the drug. Furthermore, the estimation unit may further retrain the model of the machine learning using other estimation information in predicting the estimated information. In addition, the machine learning may correspond to neural network, and an autoencoder may be used.

Further, in the prediction device, the chemical substance information of the drug may include chemical structure information and the characteristic information, the chemical structure information may be a feature value on the basis of the chemical structure of the drug, and the characteristic information may be a feature value on the basis of chemical and physical properties of the drug. Moreover, the cellular level biological information may be a feature value on the basis of information on behavior of the drug when the drug is administered to a cultured cell.

Furthermore, the pharmacological information may include a feature value of the drug on the basis of its effects on an organism and its dynamics within a body as well as the drug administration information, which amount may correspond to a feature value on the basis of a drug administration method and biological information at a time of drug administration.

Further, the output unit may predict and output a drug administration plan for the drug in predicting and outputting both the efficacy and side effects of the drug on an organism.

Moreover, the acquisition unit of the prediction device may include a crawling unit that acquires at least either the chemical substance information of the drug, the cellular level effect information of the drug, or the pharmacological information of the drug from a website present on the Internet.

A prediction method according to an embodiment is also characterized by causing a computer to execute the steps of: acquiring chemical substance information of a drug and pharmacological information of the drug; estimating estimated information of the drug by performing machine learning using the chemical substance information and the pharmacological information; and predicting and outputting both efficacy and side effect of the drug on an organism by retraining a model of the machine learning on the basis of the estimated information. Further, the acquiring step may further acquire cellular level biological information of the drug; and the estimating step may perform the machine learning using the chemical substance information, the pharmacological information, and the cellular level biological information.

In addition, a prediction program according to an embodiment is characterized by causing a computer to embody: an acquisition function of acquiring chemical substance information of a drug and pharmacological information of the drug; an estimation function of estimating estimated information of the drug by performing machine learning using the chemical substance information and the pharmacological information; and an output function of predicting and outputting both efficacy and side effect of the drug on an organism by retraining a model of the machine learning on the basis of the estimated information. Further, the acquisition function in the prediction program may further acquire cellular level biological information of the drug, and the estimation function may perform the machine learning using the chemical substance information, the pharmacological information, and the cellular level biological information.

### Advantageous Effects of the Invention

The prediction device according to the present invention includes: an acquisition unit that acquires chemical substance information of a drug and pharmacological information of the drug; an estimation unit that estimates estimated information of the drug by performing machine learning using the chemical substance information and the pharmacological information; and an output unit that predicts and outputs both efficacy and side effect of the drug on an organism by retraining a model of the machine learning on the basis of the estimated information. This integrates chemical information on compounds and information on their effects to achieve accurate and efficient creation of the desired drugs, and even prediction of the efficacy and side effects of the drugs.

In the same manner, the prediction method and prediction program also allow accurate and efficient prediction of the drug discovery of a desired drug as well as the efficacy and side effects of the drug by integrating the chemical information of a compound and its effect information.

### BRIEF DESCRIPTION OF THE DRAWINGS

Features, advantages, and technical and industrial significance of exemplary aspects of the invention will be described below with reference to the accompanying drawings, in which like numerals denote like elements, and wherein:
FIG. 1 is a schematic diagram illustrating a configuration of a prediction device and its system according to an embodiment,
FIG. 2 is a block diagram illustrating functional units of a computer,
FIG. 3 is a first schematic diagram illustrating a machine learning process,
FIG. 4 is a second schematic diagram illustrating a machine learning process,
FIG. 5 is a third schematic diagram illustrating a machine learning process,
FIG. 6 is a fourth schematic diagram illustrating a machine learning process,
FIG. 7 is a fifth schematic diagram illustrating a machine learning process,
FIG. 8 is a flowchart illustrating a prediction method according to an embodiment, and
FIG. 9 is a flowchart illustrating a prediction method according to another embodiment.

### DETAILED DESCRIPTION OF ASPECTS

One of the problems with in silico drug discovery using a computer is that the design process does not take into account the dynamics of the drug in vivo, i.e., the pharmacokinetics of the drug, or the final in vivo effects of the drug (drug efficacy and side effects: pharmacodynamics) as the final output. AI-based pharmacokinetic prediction systems called ADMET prediction (absorption, distribution, metabolism, excretion, toxicity) have begun to be developed as a method for predicting adverse drug events in recent years.

However, the predictive elements of existing prediction methods are limited, such as the affinity of a compound for a particular drug transporter or for an enzyme that has a high probability of causing cardiotoxicity upon inhibition. This is mainly due to a fact that building an AI system for ADMET prediction needs a considerable amount of experimental data, and for some items, data is insufficient. Further, the ADMET prediction and prediction of drug efficacy are independent systems, and AI system that integrates both perspectives to effectively and directly predict the final pharmacodynamics of a drug in vivo is currently absent.

One of the main challenges in developing an AI model that accurately predicts pharmacodynamics, which is the ultimate goal in drug discovery, lies in the complexity of integrating multiple biological factors. For example, a drug interacts with various drug transport and metabolism systems in vivo, influencing its pharmacokinetics. Ultimately, the drug reaches both target and non-target cells and proteins, to which it binds to varying degrees. This binding induces cellular changes that contribute to its overall pharmacological effects. To build a precise AI model, these factors need to be quantitatively and comprehensively captured and integrated with clinical information. In addition, the amount of clinical data in which efficacy and side effects are directly recorded is limited, and thus training a machine learning model using only clinical data may be recognized as difficult.

Accordingly, the prediction device, prediction method, and prediction program according to the embodiment embody a method for predicting the most critical information in drug discovery, namely the pharmacodynamics, including efficacy and side effects in vivo. This begins by extracting the structure of the drug (compound) and its chemical and physical properties, followed by gradually training cellular level effect data on stages, for example, ultimately enabling the prediction of in vivo pharmacodynamics. The prediction device, prediction method, and prediction program allow the final trained model to quantitatively predict the efficacy and side effects of an unknown candidate drug on the basis of relatively limited information such as the structure of the drug, enabling the selection of a promising candidate drug on the basis of the prediction. In addition, the output of the intermediate layers of the machine learning model can be read out so that pharmacokinetic information of candidate drugs (compounds) is acquired, which can also be used to predict the drug administration plans for the candidate drugs. Here, the candidate drugs (compounds) may be of various types including peptides, nucleic acids, and metal-containing compounds, for example, in addition to low to medium molecular weight organic compounds with molecular weights of about 100 to 5,000. Further, it may be extended to the development of macromolecular drugs such as monoclonal antibodies.

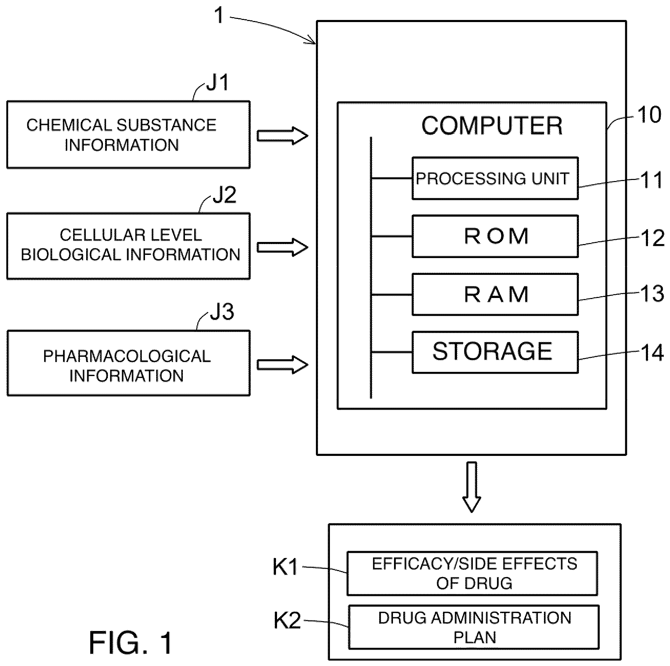

For illustrating the prediction device, prediction method, and prediction program according to an embodiment, the prediction device will be described with reference to the prediction method. A schematic diagram in FIG. 1 illustrates a configuration of a prediction device 1 according to an embodiment. The prediction device 1 is connected to an Internet connection line (not shown in FIG. 1). The prediction device 1 is capable of acquiring information published on websites opened to the public by individuals, research institutes, data centers of national, public, private, and other organizations, and medical institutions, for example, through the Internet connection line. The information to be acquired may include, for example, various information related to drug discovery, such as physical properties of compounds, biological experimental data, clinical data, and basic medical and clinical papers. In addition, information and data entered by the user of the prediction device 1 may also be used.

In FIG. 1, drug chemical substance information J1, drug cellular level biological information J2, and drug in vivo pharmacological information J3 are entered to the prediction device 1. The prediction device 1 (prediction method, prediction program) can then use machine learning trained on these pieces of information to output effective information for clinical care such as efficacy and side effects K1, and even drug administration plans K2. The drug administration plan K2 will be described below. The prediction device 1 includes a computer 10, and in terms of hardware, a processing unit 11 (e.g., GPU), ROM 12, RAM 13, storage 14, and others are implemented. The computer 10 of the prediction device 1 may be configured by various electronic computers (computing resources) such as supercomputers, mainframes, workstations, and cloud computing systems.

When the functional units of the computer 10 of the prediction device 1 in FIG. 1 are embodied by software, the computer 10 is embodied by executing the instructions of a program as software that embodies the functions. For the recording media that store this program, a "non-transient tangible media" such as a CD, DVD, semiconductor memory, and programmable logic circuit may be used. In addition, the program may be supplied to the computer 10 of the prediction device 1 via any transmission media (e.g., communication network, and broadcast wave) capable of transmitting the program.

The storage of the computer 10 of the prediction device 1 may include a storage device such as an HDD or SSD. The storage 14 may be an external server (not shown). The storage 14 stores various data, information, the prediction program, and various data needed to execute the program. In addition, the functional units that perform various computations and arithmetic operations, for example, correspond to the processing unit 11. In addition, input devices such as keyboards, and mice, for example (not shown), display units (such as displays), output devices that output data, for example, may also be appropriately connected to the prediction device 1 (computer 10).

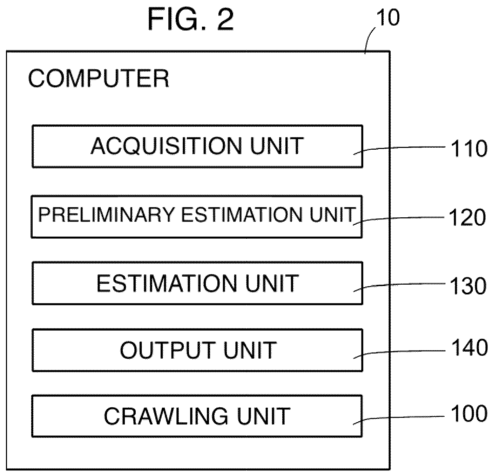

The schematic block diagram in FIG. 2 shows the functional units in the processing unit 11 of the computer 10 of the prediction device 1. The functional units may include a crawling unit 100, acquisition unit 110, preliminary estimation unit 120, estimation unit 130, and output unit 140. Processing and execution in the prediction device 1 are embodied software-wise, e.g., by a prediction program loaded into main memory.

The acquisition unit 110 acquires the drug chemical substance information J1 and drug pharmacological information J3. In addition to these, the acquisition unit 110 acquires the cellular level biological information J2 of the drug (see FIG. 1). Each of the information J1, J2, and J3 is acquired via the crawling unit 100 described below, or through input of the needed information by the user of the prediction device 1.

The chemical substance information J1 of the drug corresponds to feature values on the basis of at least the chemical structure of the drug. The information contained in the chemical substance information J1 of the drug includes chemical structure information and chemical and physical property information (characteristic information). This chemical and physical property information (characteristic information) of the drug may include, for example, the molecular weight, electric charge, LogP, pKa, hydrogen bonding properties, molecular orbitals, solubility, and membrane permeation rate of the drug (compound). In addition, it may include feature values that can be computed on the basis of at least the two-dimensional structure and/or three-dimensional structure of the compound related to the chemical structure of the drug. Examples may include feature values such as the number of atoms, and structural fragment lists, for example, which are on the basis of two-dimensional structure as well as molecular volume, molecular surface area, bond lengths, and rotational degrees of freedom, for example, which are on the basis of three-dimensional structure. Accordingly, the chemical substance information J1 is generated as feature values on the basis of its chemical structure information and characteristic information. The chemical structure information corresponds to feature values on the basis of the chemical structure of the drug. The acquisition unit 110 acquires these various features.

The cellular level biological information J2 of the drug primarily corresponds to indices that quantify the behavior and effects of the compound on cells, and is generated as feature values on the basis of the information acquired at a time when the drug is administered to cultured cells. This corresponds to information that can be acquired in vitro, and is more extensive than information acquired purely biochemically. The information may include, for example, cellular bioavailability of drug compounds (in the case of intracellular targets), as well as molecular biological information such as changes in DNA, including epigenetics, after drug administration, changes in RNA expression, and increases and decreases in transcription factors, for example, and comprehensive (omics) changes in protein expression, and intracellular metabolites, for example. The information may also include macroscopic cellular biological events (e.g., size of cells after culture, cytokine production levels, and apoptosis levels).

The pharmacological information J3 corresponds to information that affects the effects of a drug when it is administered in vivo (drug administration information) in addition to information on the effects of the drug at the in vivo level. The information on the effects of the drug at the in vivo level is acquired from clinical data (in vivo data), and specifically encompasses pharmacokinetic information in the body as well as the drug efficacy and side effects (pharmacodynamics).

The drug administration information corresponds to values that effect the pharmacokinetics, efficacy, and side effects of the drug when it is administered into the body. The pharmacokinetics, efficacy, and side effects in the body vary depending on the means of drug administration (e.g., dose, and route of administration). In addition, although clinical data do not record direct measures, the in vivo environment (e.g., target extracellular tissue information) of a diseased patient is different from that of a healthy subject, effecting the pharmacokinetic information as well as the efficacy and side effects of the drug. Accordingly, information that effects the pharmacokinetic information along with the efficacy and side effects of the drug are contained in the pharmacological information J3 as drug administration information. Specifically, the information may include the means of drug administration (e.g., dose and route of administration) and biological information at the time of drug administration (e.g., age, sex, and extracellular tissue information).

When acquiring by the acquisition unit 110, the acquisition unit 110 may include a crawling unit 100, which enables automated acquisition. The crawling unit 100 acquires at least one of the following, or two or all of them, from websites on the Internet: the drug chemical substance information J1, drug cellular level biological information J2, or drug pharmacological information J3. The website may provide information that is made publicly available from various facilities such as research institutes, national, public, private, and other data centers, and medical institutions. The acquisition by the acquisition unit 110 may use information and data acquired by the user.

In addition to chemical and physical knowledge and papers on drugs, findings and publications at the cellular and clinical levels, as well as reference values for administration continue to increase daily. Accordingly, to always acquire the latest information and data and update the machine learning model, the computer 10 of the prediction device 1 acquires the public information on the website through the crawling unit 100 and stores it in the storage 14. In addition, the user of the prediction device 1 may allow the storage 14 to store the information and data.

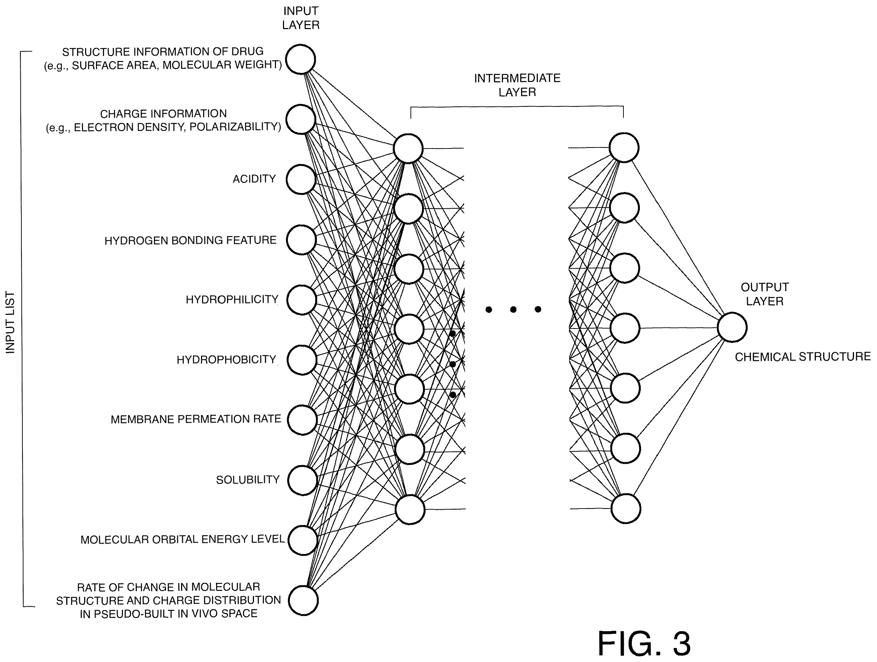

The preliminary estimation unit 120 preliminarily estimates the chemical structure of the drug on the basis of the characteristic information of the drug (see FIG. 3). The acquisition of the characteristic information of the drug is either through the crawling unit 100 or by input of the needed information by the user of the prediction device 1.

The preliminary estimation unit 120 serves to preliminarily estimate the chemical structure of the drug in the estimation unit 130 described below on the basis of the characteristic information. Specifically, the characteristic information may include: for example, chemical structure information including surface area and molecular weight of the drug compound; charge information including electron density and polarizability; acidity; hydrogen bonding properties; hydrophilicity; hydrophobicity; energy level of molecular orbital; membrane permeation rate; and solubility; as well as rate of change in the chemical structure and characteristic information of the drug under certain environments, such as the rate of change in molecular structure and charge distribution in the case of interacting with polar groups such as amino acids and phosphoric acids and non-polar groups such as alkyl and phenyl groups. In addition, the chemical substance information includes, along with the characteristic information, chemical structure information, i.e., feature values that can be computed on the basis of at least the two-dimensional and three-dimensional structures of the compound related to the chemical structure of the drug. Examples include feature values on the basis of the two-dimensional structure, such as the number of atoms and a structural fragment list as well as those on the basis of three-dimensional structure such as the molecular volume, molecular surface area, bond length, and rotational degrees of freedom.

In the schematic diagram of the machine learning process in FIG. 3, feature values on the basis of the chemical and physical properties of the drug (drug characteristic information) are incorporated into the input layer in FIG. 3 at the time of selecting the molecular structure of the drug. The incorporation of machine learning in the preliminary estimation unit 120 is optional, depending on the process, such as when the molecular structure of the drug needs to be selected.

On the basis of the combination of input values, the chemical structure of the drug is output by machine learning. The output format may include, for example, expression by simplified molecular input line entry system (SMILES) notation. For example, neural networks are used for machine learning in the preliminary estimation unit 120, estimation unit 130, and output unit 140.

Here, when the machine learning is executed in the processing unit, machine learning using, for example, neural networks is executed. Other machine learning methods may include association rule learning, random forests, and support vector machines. On the information acquired through the crawling unit 100, data processing using programming languages and natural language processing may be performed.

If the prediction accuracy does not reach the target value after training using the above input items, the autoencoder or similar device is trained again using the chemical structure, and additional latent feature values are extracted to compensate for the parts that have not been fully predicted. The automatically extracted features are added to the input value items as input values, and the machine learning model is retrained to improve the prediction accuracy. As described above, preliminary network training in machine learning is performed.

The estimation unit 130 estimates the estimated information of the drug by performing machine learning using the chemical substance information J1 and pharmacological information J3. Further, the estimation unit 130 estimates the estimated information of the drug by performing machine learning using the chemical substance information J1 and pharmacological information J3 along with the cellular level biological information J2. To increase the accuracy of machine learning in the output unit 140, it is desirable to increase the pharmacokinetic and cellular level biological information as well as the estimated information in addition to the characteristic information as in the embodiment. In addition, the estimation unit 130 estimates the characteristic information of the drug on the basis of the chemical structure contained in the chemical substance information J1 and the drug administration information contained in the pharmacological information J3, estimates the pharmacokinetic information of the drug on the basis of the chemical structure contained in the chemical substance information J1 and the drug administration information contained in the pharmacological information J3, and estimates the biological information J2 on the basis of the chemical structure contained in the chemical substance information J1 and the drug administration information contained in the pharmacological information J3. In addition, the estimation unit 130 further retrains the machine learning model using other estimated information at the time of prediction of the estimated information.

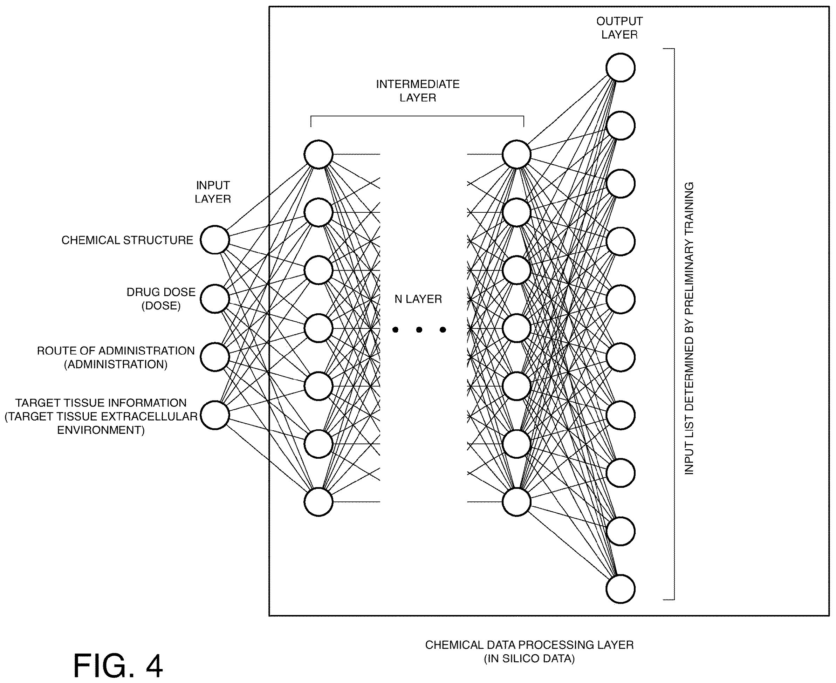

The schematic diagram of the machine learning process in FIG. 4 shows the process of outputting the characteristic information of the drug from the limited chemical substance information of the drug. Specifically, the chemical structure of the drug is input, and the feature value of each item of the characteristic information of the drug is output. In this step, as shown in the schematic diagram in FIG. 4, feature values of the drug administration information such as drug dose, route of administration, and target tissue information may be added to the input values.

In the stage of the machine learning process in FIG. 4, the machine learning model is trained to compute the same output values even when the feature values of the drug administration information in the input layer are changed as needed. The reason for this is that the goal of this stage is to extract feature values on the basis of the pure chemical and physical properties of the drug, and to allow the machine learning model to learn input-output relationships that are independent of the input values such as the drug dose, route of administration, and target tissue information. The network in the schematic diagram in FIG. 4, unlike the preliminary network in FIG. 3, has its weights randomly initialized.

As shown in the schematic diagram of the machine learning model in FIG. 4, a perspective in consideration of the drug administration to the organism, such as drug dose, route of administration, and target tissue information is added to the machine learning that has previously associated only the chemical structure (chemical substance information) with the characteristic information of the drug. This thereafter trains the machine learning model in consideration of not only the chemical structure but also the drug administration to the organism to improve the prediction accuracy.

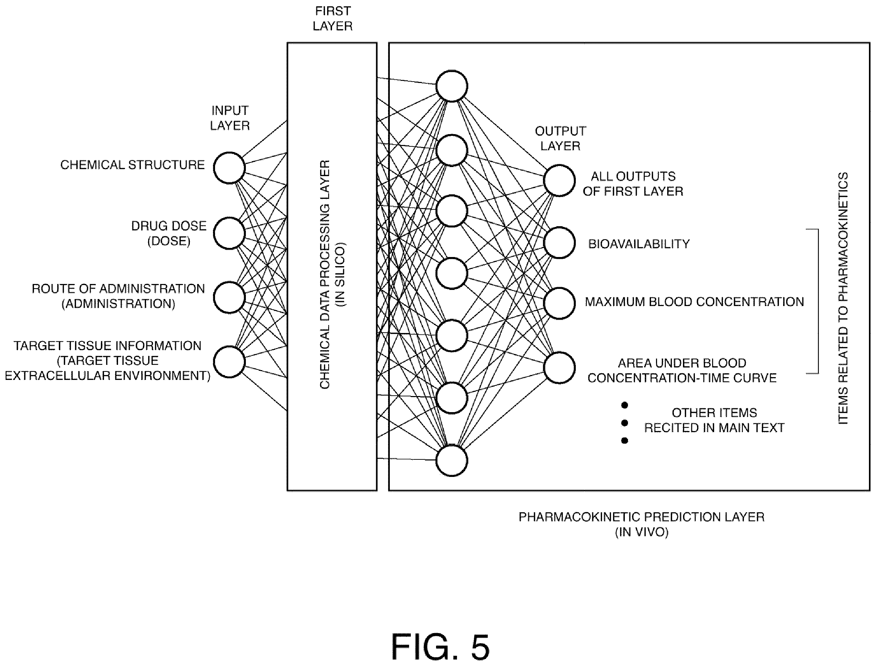

To improve the prediction accuracy of the output unit, a machine learning model may then be trained to estimate the pharmacokinetics of the drug in vivo using the chemical substance information and pharmacological information of the drug as inputs. In this step, when the machine learning model in FIG. 4 is retrained, the chemical substance information needed for the input data is directed to the chemical structure only. Also, as shown in the schematic diagram in FIG. 5, the output layer generates not only items related to the pharmacokinetics of the drug (feature values on the basis of the information on the behavior of the drug when it is administered to the organism) but also the same items as the outputs from the chemical data processing layer (in silico) in FIG. 4 above.

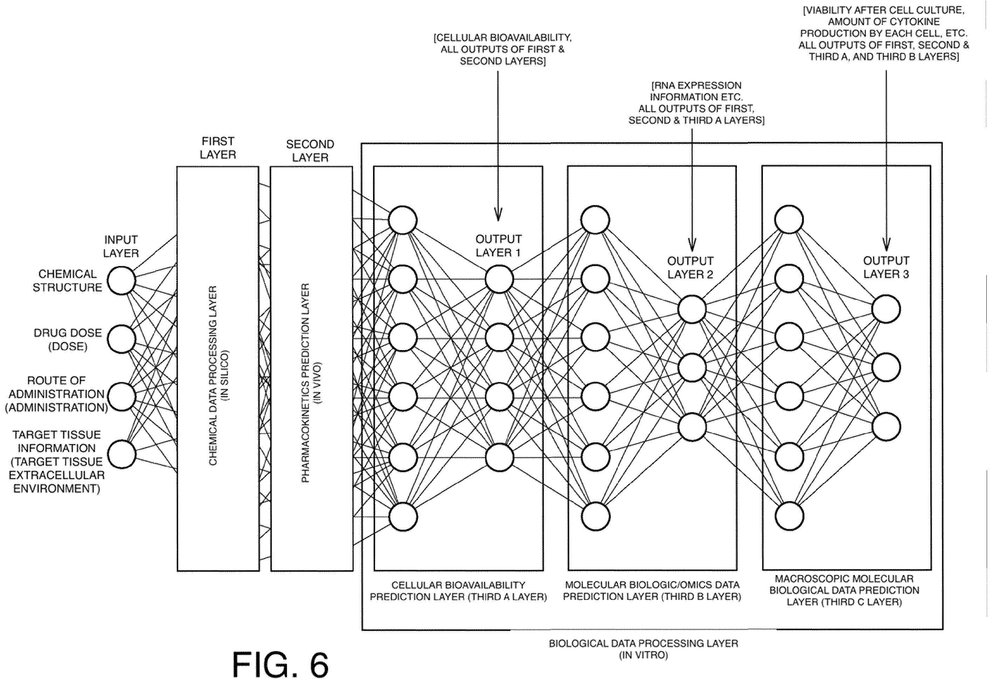

The reason for this is that when performing transfer learning on the machine learning model trained to output the pharmacokinetics as in FIG. 6 below, the first layer outputs (characteristic information) are also directly used to facilitate prediction of outputs. To accommodate changes in the characteristic information of the drug under certain environmental conditions (e.g., under acidic conditions), unlike the output in FIG. 4 above, the characteristic information to be output is computed through retraining on the changes in the in silico output items caused by the target tissue information, which is one of the input values. After this retraining, the trainable parameters of all or some units in the first layer may be frozen so that their values may fail to be updated during the subsequent training.

In the training for estimating the pharmacokinetics, as shown in the schematic diagram in FIG. 5, on the basis of the input values of the "chemical substance information (chemical structure) of the drug," "drug dose," and "route of administration," the feature values related to the pharmacokinetics such as "bioavailability (an index of how much of the administered drug reaches the blood)," "maximum blood concentration," "area under the blood concentration-time curve," "volume of distribution," "renal clearance," and "hepatic metabolic rate" are output as the feature values on the basis of the information on the behavior of the drug when it is administered to the organism. Here, the output values related to the pharmacokinetics are trained so that they remain constant independent from the input values that are not involved in the pharmacokinetics (target tissue information in FIG. 5).

As for the bioavailability, it is trained in terms of how the bioavailability varies depending on the route of administration of drug compound. The "route of administration" item is entered as 0 (intravenous), 1 (oral), and 2 (rectal), for example. When the intravenous is entered, for example, the bioavailability is 100%, but for other routes, for example, 1% for oral, and 50% for rectal are output.

Training of the machine learning model may then be performed on multiple pieces of cellular level biological information of the drug (i.e., in vitro data) to further improve the prediction accuracy of the output unit 140. This is classified into three broad categories here: "cellular bioavailability," "intermediate data including molecular biological data," and "macroscopic biological data." These correspond to feature items on the basis of the information on the behavior of the drug when it is administered to the cultured cells. The order of this training is on the basis of the biological rationale; for example, the RNA expression is trained before the protein expression. The order of the training is allowed to be swapped, and not all stages are needed in the machine learning.

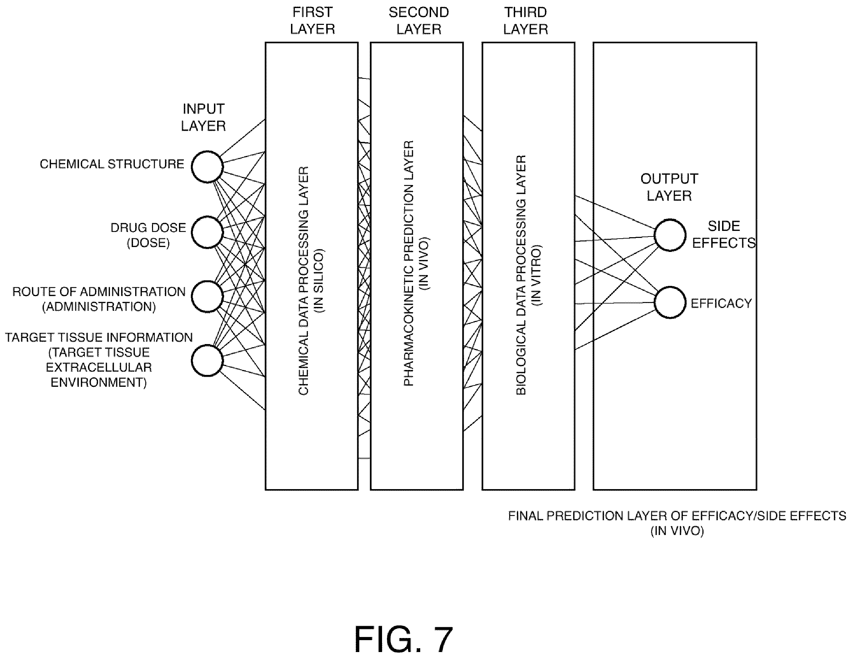

The main purpose of training with multiple pieces of the cellular level biological information (in vitro data) is to reduce the amount of data needed for training the next output layer (see the final in vivo prediction layer of the efficacy/side effects in FIG. 7). Accordingly, the training content is adjusted as appropriate so that the final prediction of the efficacy and side effects by the machine learning model reaches a sufficient level of accuracy.

Again, the chemical substance information and pharmacological information of the drug are used as inputs to estimate the cellular level biological information of the drug, i.e., the behavior of the drug when it is administered to the cultured cells. The schematic diagram of the machine learning process in FIG. 6 shows the process of retraining the machine learning model for estimating the characteristic and pharmacokinetic information in FIG. 5 above to output the cellular level biological information.

Obviously, in the feature values that correspond to the cellular level biological information of the drug (in vitro data), the amount of the drug added in culture is basically not consistent with the drug dose item. Accordingly, the drug dose corresponding to each data may be estimated in advance by the following method. For example, after training of the model shown in the schematic diagram in FIG. 5 is completed, the chemical structure, route of administration, and target tissue information used during the in vitro data acquisition are fixed. The dose (drug dose) value is then varied and the resulting changes in the values of the pharmacokinetic information are examined. Next, a dose is sought such that the concentration of the drug in the culture solution used in the in vitro system is the same as the extracellular fluid concentration of the drug in the tissue as estimated by the predictive pharmacokinetic models. At that time, the composition information of the culture solution is input as the fixed target tissue information. The drug dose is estimated independently for 0: intravenous, 1: oral, and 2: rectal, for example, since the value of the pharmacokinetic information varies depending on the route of administration.

The cellular bioavailability in the third a layer of the schematic diagram in FIG. 6 corresponds to the value of how much of the drug compound enters different cells, such as the amount of drug in the cell/amount of drug in the culture solution. The cellular bioavailability is measured under various administration doses/culture conditions (target tissue information) for a wide variety of cells over a specific culture period. In addition to the cellular bioavailability, the first layer output "characteristic information" and the second layer output "pharmacokinetic information" under the conditions of the target tissue information are also output.

In the third b layer of the schematic diagram in FIG. 6, molecular biology data, data of comprehensive (omics) changes, such as changes in protein expression, are acquired. Examples may include RNA expression in cells, and expression of transcription factors, for example. RNA and other expression information of various cells measured at specific culture periods and under various doses/culture conditions (target tissue information) are used for training. These trainings are performed after the cellular bioavailability training in the layer 3a.

In the third c layer of the schematic diagram in FIG. 6, macroscopic biological features are used as multiple variation features (in vitro data). For example, training is performed using cell size after culture, viability after cell culture, protein expression inside and outside the cell that can be obtained by measurements such as flow cytometry, and cytokine production level by each cell, for example, as output values.

The output unit 140 retrains the machine learning model to predict and output the efficacy and side effects of the drug in the organism performing machine learning with the estimated information from the estimation unit 130 as the input. The output may be a prediction of either efficacy or side effects depending on the setting.

The process in the output unit 140 is that on the basis of all of the output values of each layer so far, the efficacy and side effects K1 of the drug as a main objective, as well as the drug administration plan K2 as information associated with the efficacy and side effects are output (see FIG. 1). In the schematic diagram in FIG. 7, the machine learning model trained in the estimation unit 130 is retrained using the clinical data to output the efficacy and side effects of the drug. The clinical data is acquired from all or part of the data published on the website. The crawling unit 100 described above is used for the acquisition.

In the same manner as in the previous machine learning processes, the input values correspond to the chemical structure of the drug and the drug administration information. In the schematic diagram in FIG. 7, "drug dose," "route of administration," and "target tissue information" are used. The drug dose may be a dose of the drug compound per administration, or it may be a total dose for the entire period of its administration. For the "target tissue information," information on the extracellular environment of the tissue that can be estimated from the primary symptoms of the disease is entered. Since this data cannot be obtained from the published clinical trials, it may be set to physiological values for convenience. However, the target disease may cause changes in the extra-tissue fluid of the target tissue. For example, in the case where severe inflammation occurs in tissues, the external environment also varies depending on the severity of the inflammation, and thus the machine learning model is constructed with a certain degree of flexibility to account for these variations. Other factors such as age and medical history can be taken into account in the model, but as shown in FIG. 7, the model can also be trained without taking these factors into account. Although this poses potential issues, in the same manner as in the dosing periods, these factors are excluded in the machine learning model in order to secure a larger amount of data.

As one of the outputs, the ratio of "people with confirmed efficacy (or side effects)" to "the number of people participating in clinical trials" is used. The direct meaning of "efficacy" is directed to the rate of remission of the disease. However, if that value is focused, a complete picture of the effects of the drug compound on the body cannot be easily acquired. The output values therefore include data that are indirect indicators of disease remission, such as those obtained from blood tests, for example, which are usually performed in clinical trials. Taking rheumatoid arthritis as an example, in addition to the data of "remission rate of rheumatoid arthritis," test results such as CRP as an inflammatory marker, which is usually tested, are added as an item called "reduction rate of CRP." In addition, the side effect outputs may be presented as numerical indicators that suggest the likelihood of occurrence per individual, such as "incidence of headache after taking the drug: 80%," "rash: 50%," and "probability of abnormal hepatobiliary marker values: 20%."

As described above, drugs used for "rheumatoid arthritis" but not for "systemic lupus erythematosus" are consistent in terms of the CRP reduction, and both can be interpreted as drugs that "reduce inflammation." For side effects, both subjective items such as "headache" and objectively obtainable items such as blood markers including renal markers can be used as in the previous examples. Items such as "decrease in blood pressure," which corresponds to efficacy if it is an antihypertensive drug and also a side effect if it is an anaphylactic shock, are not unified, and separate items are provided: "decrease in blood pressure" as efficacy and "decrease in blood pressure" as a side effect.

Because of the wide variety of drug efficacy/side effect items, items with missing values may be concerned in the data for each drug in the clinical database. To supplement the missing values, the drug data in the clinical database are input into the trained machine learning model (machine learning model in the estimation unit) at this point of time, and correlation coefficients among all the drugs are computed on the basis of all or some of the output values, which are then used to create similarity indices among the drugs. If an item with a missing value is identified for a certain drug (compound), the missing value can be supplemented by the effects of another drug (compound) listed in the clinical database that has a high correlation to the certain drug (compound).

From the above description, the prediction device 1 (its prediction method and prediction program) allows, from the chemical structure of a candidate drug, prediction by simulation, through machine learning, for either the efficacy or side effects, or both efficacy and side effects, corresponding to the chemical structure of the candidate drug. That is, the prediction device 1 (its prediction method and prediction program) causes the machine learning to learn the chemical information (chemical substance information of a drug), biological information (cellular level biological information of a drug for cells), and clinical information (pharmacological information of a drug to the organism), allowing prediction of not only the chemical properties of a drug but also its effects at the cellular level as well as its efficacy and side effects in vivo on the basis of just the information on the chemical structure of the drug. Further, pharmacokinetic values are also computed in the process so that the drug administration plan for the candidate drug can also be predicted. The drug administration plan here refers to the route of administration, frequency of administration, and dose. Also, even for conditions for which no known cure is currently present, the drug efficacy can be estimated to a certain degree, along with the side effects of the candidate drug from information such as the cellular level biological information and blood markers. As described above, the chemical structure of a candidate drug with potential efficacy can be estimated without actually synthesizing the candidate drug, and further, the efficacy, side effects, and administration plan of the candidate drug can be predicted. This can contribute greatly to simplifying the screening process in new drug development (drug discovery) and selecting the direction of candidate drug synthesis.

An example of drug discovery on the basis of the prediction of efficacy and side effects of an unknown drug using trained machine learning models with the prediction device 1 is summarized below.

Initially, compounds are randomly selected to create a list of candidate drugs covering a wide variety of compounds.

At this time, compounds that are difficult to synthesize according to the existing synthetic difficulty evaluation methods or other methods are excluded from the List-1.

All types of compounds are then entered into the trained network of the machine learning model to obtain the remission rate (efficacy) of the disease for which a cure is desired and side effects for each compound.

The obtained efficacy and side effects are compared with the reference values, drugs that fall below the reference for efficacy or exceed the reference for side effects are excluded, and a List-2 is generated with the remaining drug candidates.

A considerable number of drugs are then generated by modifying a part of the chemical structure of each drug in List-2, drugs that are difficult to synthesize are excluded, the efficacy and side effects of the remaining drugs are predicted, drugs that do not meet the reference values are excluded, and a List-3 is generated with the remaining drugs.

The evaluation will be performed in the same manner with some modification to the newly added compounds in List-3. This process of modifying and evaluating compounds is repeated, optimization of the drug efficacy and side effects is iteratively attempted, and the iterative process is terminated when no significant improvement is recognized in the efficacy or side effects for any of the drugs on the list. Finally, the drugs on the list are ranked, and the top-ranking drugs are selected as the final candidate drugs.

It is considered appropriate to give priority to the entry of information such as 0 (intravenous) in the route of drug administration. However, no value has been established for dose. For this, a process is performed such that the values are varied as appropriate, and the changes in the efficacy and side effects in accordance with the variation of the values are verified to determine a realistic dose value range, and only the drugs with the efficacy that exceeds the reference efficacy and side effect that is below the reference side effect are kept on the list as drug candidates. In addition, efficacy and side effects can be evaluated by changing the conditions of the route of drug administration according to the purpose in the case where an oral drug is needed in addition to intravenous administration, for example.

In the case of drug discovery on the basis of the prediction of the efficacy and side effects of unknown drugs using a trained machine learning model using the prediction device 1 according to the embodiment, multiple candidate drugs can be organized in the order of degree of efficacy and side effects. This facilitates the determination of the order of priority which of the multiple candidate drugs should be selected for each phase of the trials. Since the efficacy and side effects of candidate drugs can be predicted in advance, a priority ranking can be assigned to candidate drugs to improve the efficiency of planning and management of regulatory trials, for example.

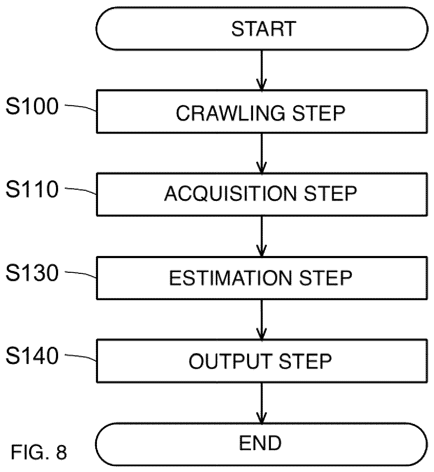

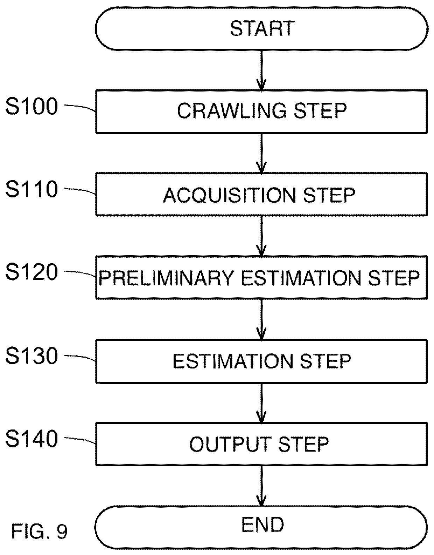

With reference to flowcharts in FIGS. 8 and 9, a prediction method as well as a prediction program in the computer 10 of the prediction device 1 will be described. The prediction method is performed by the processing unit 11 of the computer on the basis of the prediction program. The prediction program causes the computer 10 in FIG. 1 and FIG. 2 to perform crawling, acquisition, preliminary estimation, estimation, output, and other functions. Since the description of the functions is redundant with the description of the prediction device 1 above, the details thereof are omitted.

As shown in the flowchart in FIG. 8, the processing of the processing unit 11 of the computer 10 has the following steps: crawling step (step S100), acquisition step (step S110), estimation step (step S130), and output step (step S140). The steps needed for the computer 10 itself to operate are naturally included. In the flowchart shown in FIG. 8, the crawling step (step S100) may be omitted. As described above, the crawling step (step S100) can be omitted if the user of the prediction device 1 manually enters information and data.

The crawling function acquires at least one of the following from a website present on the Internet (step S100; crawling step): chemical substance information, cellular level biological information of the drug for the cells, or pharmacological information of the drug. The acquisition function acquires the chemical substance information of the drug and the pharmacological information of the drug (step S110; acquisition step). The acquisition function can also acquire the chemical substance information of the drug, pharmacological information of the drug, and cellular level biological information of the drug. The estimation function estimates the estimated information of the drug by performing machine learning using the chemical substance information and pharmacological information (step S130; estimation step). The estimation function can also estimate estimated information of the drug by performing machine learning using the chemical substance information, pharmacological information, and cellular level biological information. The output function predicts and outputs both the efficacy and side effects of the drug on the organism by retraining the machine learning model on the basis of the estimated information (step S140; output step).

With regard to the prediction method and prediction program according to another embodiment in the computer 10 of the prediction device 1, the flowchart in FIG. 9 shows that the processing of the processing unit 11 of the computer 10 includes the following steps: crawling step (step S100), acquisition step (step S110), preliminary estimation step (step S120), estimation step (step S130), and output step (step S140). The steps needed for the computer 10 itself to operate are naturally included. Here, a preliminary estimation function will be included. The preliminary estimation function preliminarily estimates the chemical structure of the drug on the basis of the characteristic information (step S120; preliminary estimation step). In the flowchart shown in FIG. 9, the crawling step (step S100) may be omitted. As described above, the crawling step (step S100) can be omitted if the information and data are entered by the user of the prediction device 1.

The computer program of the present invention described above may be recorded on a recording medium readable by a processor, and the recording medium may correspond to a "non-transitory tangible medium" such as a disk, card, semiconductor memory, or programmable logic circuit.

The computer program above can be implemented using, for example, scripting languages such as ActionScript and JavaScript (registered trademark), object-oriented programming languages such as Objective-C and Java (registered trademark), and markup languages such as HTML5.

### Description of Reference Numerals

1: Prediction Device
10: Computer
11: Processing Unit
12: ROM
13: RAM
14: Storage
100: Crawling Unit
110: Acquisition Unit
120: Preliminary Estimation Unit
130: Estimation Unit
140: Output Unit
J1: Drug Chemical Substance Information
J2: Cellular Level Biological Information of Drugs
J3: Pharmacological Information of Drugs
K1: Efficacy and Side Effects of Drugs
K2: Drug Administration Plan

## Claims

1. A prediction device comprising:
an acquisition unit that acquires chemical substance information of a drug and pharmacological information of the drug;
an estimation unit that estimates estimated information of the drug by performing machine learning using the chemical substance information and the pharmacological information; and
an output unit that predicts and outputs both efficacy and side effect of the drug on an organism by retraining a model of the machine learning on the basis of the estimated information.

2. The prediction device according to claim 1, wherein
the acquisition unit further acquires cellular level biological information of the drug, and
the estimation unit performs the machine learning using the chemical substance information, the pharmacological information, and the cellular level biological information.

3. The prediction device according to claim 1, wherein the estimation unit estimates characteristic information of the drug on the basis of a chemical structure included in the chemical substance information and drug administration information included in the pharmacological information.

4. The prediction device according to claim 1, wherein the estimation unit estimates pharmacokinetic information of the drug on the basis of a chemical structure included in the chemical substance information and drug administration information included in the pharmacological information.

5. The prediction device according to claim 2, wherein the estimation unit estimates the cellular level biological information on the basis of a chemical structure included in the chemical substance information and drug administration information included in the pharmacological information.

6. The prediction device according to claim 3, further comprising a preliminary estimation unit that preliminarily estimates the chemical structure of the drug on the basis of the characteristic information of the drug.

7. The prediction device according to claim 1, wherein the estimation unit further retrains the model of the machine learning using other estimation information in predicting the estimated information.

8. The prediction device according to claim 1, wherein the machine learning is a neural network and an autoencoder is used.

9. The prediction device according to claim 3, wherein the chemical substance information of the drug includes chemical structure information and the characteristic information, the chemical structure information is a feature value on the basis of the chemical structure of the drug, and the characteristic information is a feature value on the basis of chemical and physical properties of the drug.

10. The prediction device according to claim 2, wherein the cellular level biological information is a feature value on the basis of information on behavior of the drug when the drug is administered to a cultured cell.

11. The prediction device according to claim 3, wherein the pharmacological information includes a feature value of the drug on the basis of its effect on an organism and its dynamics within a body as well as the drug administration information, which amount corresponds to a feature value on the basis of a drug administration method and biological information at a time of drug administration.

12. The prediction device according to claim 1, wherein the output unit predicts and outputs a drug administration plan for the drug in predicting and outputting both the efficacy and side effects of the drug on an organism.

13. The prediction device according to claim 2, wherein the acquisition unit includes a crawling unit that acquires at least either the chemical substance information of the drug, the cellular level biological information of the drug, or the pharmacological information of the drug from a website present on the Internet.

14. A prediction method causing a computer to execute the steps of:
acquiring chemical substance information of a drug and pharmacological information of the drug;
estimating estimated information of the drug by performing machine learning using the chemical substance information and the pharmacological information; and
predicting and outputting both efficacy and side effect of the drug on an organism by retraining a model of the machine learning on the basis of the estimated information.

15. The prediction method according to claim 14, wherein
the acquiring step further acquires cellular level biological information of the drug; and
the estimating step performs the machine learning using the chemical substance information, the pharmacological information, and the cellular level biological information.

16. A prediction program causing a computer to embody:
an acquisition function of acquiring chemical substance information of a drug and pharmacological information of the drug;
an estimation function of estimating estimated information of the drug by performing machine learning using the chemical substance information and the pharmacological information; and
an output function of predicting and outputting both efficacy and side effect of the drug on an organism by retraining a model of the machine learning on the basis of the estimated information.

17. The prediction program according to claim 16, wherein
the acquisition function further acquires cellular level biological information of the drug, and
the estimation function performs the machine learning using the chemical substance information, the pharmacological information, and the cellular level biological information.
